# EUROPEAN PATENT SPECIFICATION

(11) **EP 4 192 809 B1**
(45) Date of publication and mention of the grant of the patent: **03.09.2025**
(21) Application number: 21749709.8
(22) Date of filing: 30.07.2021
(51) Int. Cl.: C07C 249/08, C07C 249/12, C07C 251/52

(54) **PROCESS FOR THE PREPARATION OF A KEY INTERMEDIATE OF SIPONIMOD**
VERFAHREN ZUR HERSTELLUNG EINES SCHLÜSSELZWISCHENPRODUKTS VON SIPONIMOD
PROCÉDÉ DE PRÉPARATION D'UN INTERMÉDIAIRE CLÉ DE SIPONIMOD

(30) Priority: 10.08.2020 IT 202000019897
(43) Date of publication of application: 14.06.2023
(73) Proprietor: OLON S.p.A., 20053 Rodano (MI) (IT)
(72) Inventor: VALLI, Matteo, 20053 Rodano (MI) (IT); SADA, Mara, 20053 Rodano (MI) (IT); FELICIANI, Lazzaro, 20053 Rodano (MI) (IT); BERTOLINI, Giorgio, 20053 Rodano (MI) (IT)
(74) Representative: Bottero, Carlo
(86) International application number: PCT/IB2021/056956
(87) International publication number: WO 2022/034426

(56) References cited:
- WO-A1-2019/064184
- DATABASE 20171210 [online] 10 December 2017 (2017-12-10), "Benzenmethanol, 4-bromo-3-(trifluoromethyl)-1-(4-methylbenzenesulfonate)", XP002802726, Database accession no. CHEMCATS
- DATABASE REGISTRY [online] CHEMICAL ABSTRACTS SERVICE, COLUMBUS, OHIO, US; 10 December 2017 (2017-12-10), XP002802726, retrieved from STN Database accession no. RN-2154989-03-2
- MALACHOWSKI WILLIAM P ET AL: "O-alkylhydroxylamines as rationally-designed mechanism-based inhibitors of indoleamine 2,3-dioxygenase-1", EUROPEAN JOURNAL OF MEDICINAL CHEMISTRY, ELSEVIER, AMSTERDAM, NL, vol. 108, 17 December 2015 (2015-12-17), pages 564 - 576, XP029383533, ISSN: 0223-5234, DOI: 10.1016/J.EJMECH.2015.12.028

## Description

### Summary of the Invention

The present invention relates to a process for the preparation of a key intermediate and other intermediates useful for the synthesis of Siponimod, a drug used for the treatment of multiple sclerosis. Object of the invention are also said novel intermediates.

### Background art

Siponimod is the international common designation of 1-[[4-[(1E)-N-[[4-cyclohexyl-3-(trifluoromethyl)phenyl]methoxy]C-methylcarbonimidoil]-2-ethylphenyl]methyl]azetidin-3-carboxylic acid having the following formula:

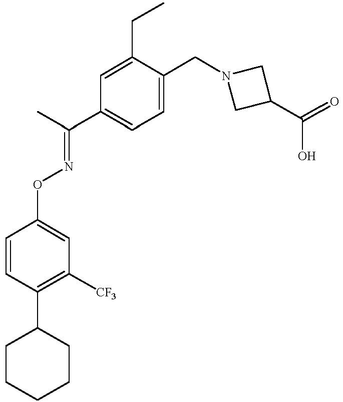

Siponimod is the active ingredient in Mayzent^{®}, a drug developed by Novartis and approved in several countries for the treatment of multiple sclerosis.

Siponimod and its synthetic route were first described and claimed in the Patent Application WO2004103306 filed by IRM LCC.

Subsequently, the Patent Application WO2013113915A1 was filed by Novartis describing the synthesis of Siponimod hemifumarate in six steps from differently substituted trifluorotoluene in the ortho position, as shown in the following scheme:

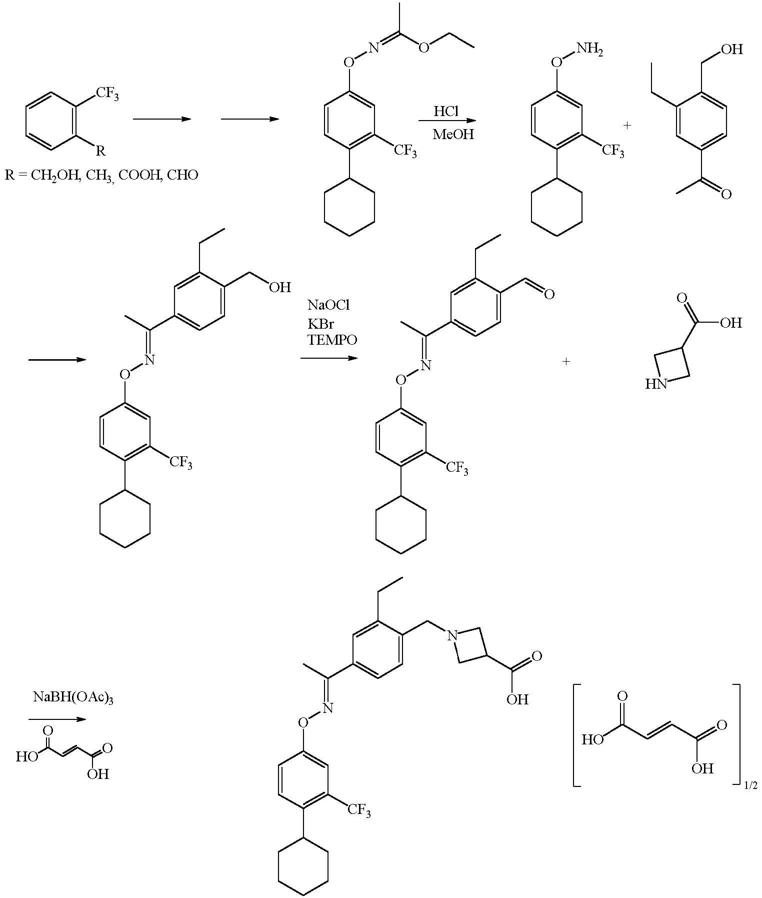

In the subsequent Patent Application WO2019064184A1 by Dr. Reddy's Laboratories Limited, the nine-step synthesis of Siponimod from 4-bromo-3-trifluoromethylbenzoic acid is described as shown in the following scheme:

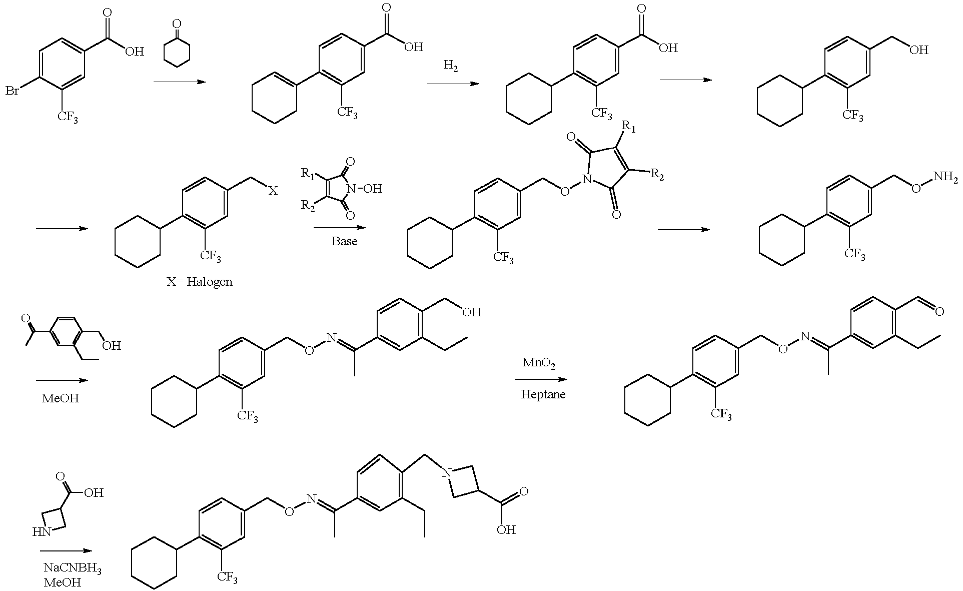

However, the process reported requires several purification steps on chromatographic column, with a consequent lowering of the total yield; the overall yield of the process is in fact about 3.5%. It is clear that this yield is incompatible with competitive large-scale production.

Therefore, there is a need to find novel industrially applicable processes that allow Siponimod to be obtained with good yields, by performing a minimum number of steps and without excessive use of solvents.

### Objects of the invention

A purpose of the invention is to provide a novel process for the preparation of intermediates useful in the synthesis of Siponimod that is industrially scalable and leads to the desired product, in high yields, with a minimum number of steps and without wasting solvents.

Another purpose of the present invention is to provide novel synthetic intermediates useful in the preparation of Siponimod and other molecules having similar structure.

### Description of the invention

The Applicant has developed a novel process which involves few purification and isolation treatments of the intermediates and which allows to obtain with good yields the key intermediate 4-[1-[(*E*)-4-cyclohexyl-3-trifluoromethyl-benzyloxy-imino]-ethyl)-2-ethyl-benzaldehyde (I), precursor of Siponimod. Said intermediate can then be directly transformed into Siponimod via a reductive amination reaction known from the literature.

In an attempt to simplify the synthesis of Siponimod and to improve its yields, an attempt was made to introduce the cyclohexyl group into the molecule through an organometallic coupling reaction on a different synthesis intermediate. However, experimental tests have shown that the introduction of the cyclohexyl group cannot be successfully carried out on any intermediate even though, in theory, nothing would preclude it. An example of such an unsuccessful synthesis is shown in the Experimental Section of the present invention.

Surprisingly, during these tests it was observed that the introduction of the cyclohexyl group on the phenyl ring in the final steps of the process, instead of in the initial steps as described in the prior art, allows the key intermediate (I) to be obtained without the need to purify and/or isolate the reaction intermediates (e.g. by chromatographic column as described in the prior art) resulting in an increased reaction yield. Thus, this different synthetic approach has made it possible to accomplish an industrially scalable and economically and ecologically advantageous process, since it does not require the use of large quantities of solvents.

Object of the invention, according to one aspect thereof, is the preparation of the compound of formula (I), a key intermediate in the synthesis of Siponimod, according to the following scheme:

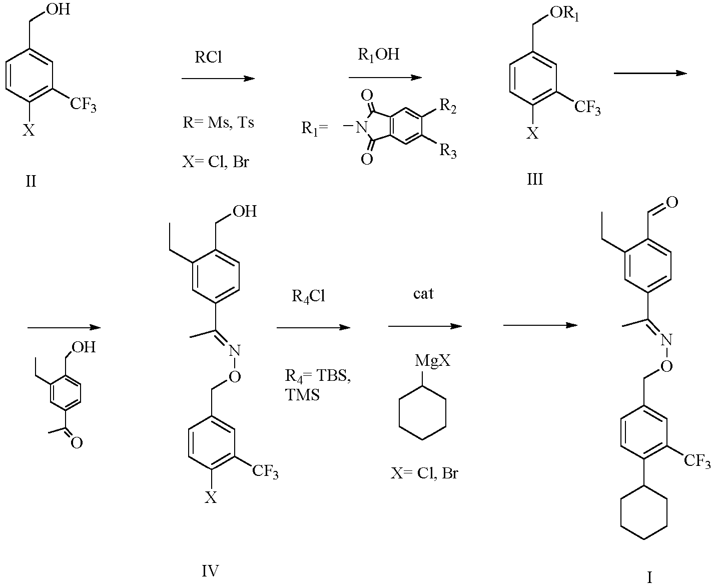

More particularly, object of the invention is a process for the preparation of the compound of formula (I) 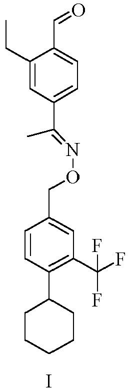 comprising the following steps:
a) converting the compound of formula (II) into the compound of formula (III), passing through the formation of the intermediate (IIa) according to the following scheme: 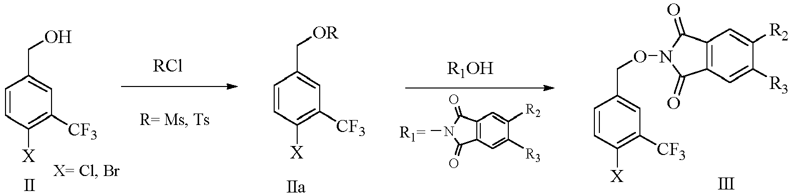 wherein R is selected from mesyl and tosyl and R₁ is a group of formula 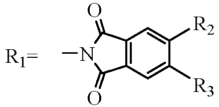
   wherein R₂ and R₃, each independently, represent hydrogen, C1-C4-alkyl, halogen, nitro or R₂ and R₃ together form an aliphatic or aromatic, 5 to 7 membered ring,
   in suitable solvents;
b) converting the thus obtained compound of formula (III) into the compound of formula (IV) by the hydrolysis of the R₁ group and the subsequent reaction with 3-ethyl-2-hydroxymethylacetophenone, passing through the formation of the intermediate (IIIa) according to the following scheme 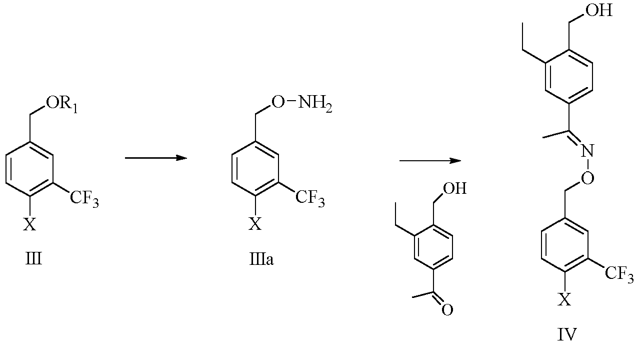 in suitable solvents;
c) converting the compound of formula (IV) into the compound of formula (I), in the presence of metal catalysts and an oxidizing agent, upon the protection of the hydroxy group, introduction of the cyclohexyl group and deprotection of the hydroxy group, passing through the formation of the compounds (IVa), (IVb), (IVc) according to the following scheme 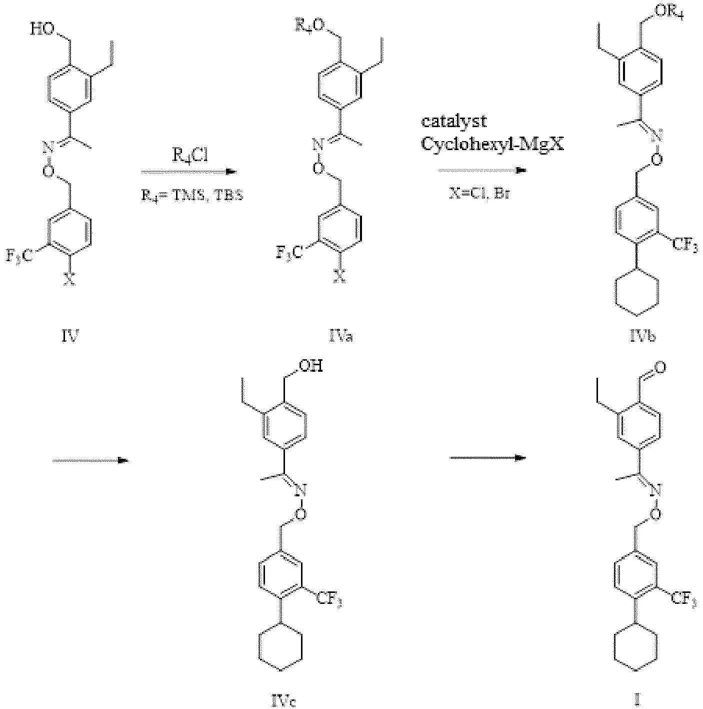
   wherein R₄ is selected from a protecting group of the hydroxy function, in suitable solvents;
   said process comprising that some or all of the compounds of formula (IIa), (IIIa), (IVa), (IVb) and (IVc) are not isolated from the reaction crude and/or purified.

According to an embodiment, none of the compounds of formula (IIa), (IIIa), (IVa), (IVb) and (IVc) is isolated from the reaction crude and/or purified.

In fact, it has been observed that the compounds (IIa), (IIIa), (IVa), (IVb) and (IVc) are intermediates that do not need isolation and purification in order to obtain the desired compound of formula (I), and that their transformation into the next intermediate can take place directly on the crude residue of the reaction that generated them. Nevertheless, if desired or necessary, some or all of said intermediates may be isolated for, for example but not limited to, analytical evaluations or for use of said compounds in syntheses other than the present one.

Therefore, the process of the present invention is readily realizable in an industrial setting, is ecologically cost-effective and provides high yields and purity.

The starting compound of formula (II) is known in the art, as well as the reactants used for the reactions of the process described herein.

In step (a), the 4-chloro-3-trifluoromethylbenzyl alcohol (II) is converted into the compound (III) by activating the hydroxyl group with an activating agent selected from mesylchloride or tosylchloride, preferably mesylchloride and subsequently by reacting with an N-hydroxy-imide.

The activation reaction to give the compound (IIa) is carried out in a suitable solvent, for example in ethers, preferably in solvents selected from tetrahydrofuran, methyl-tetrahydrofuran , dioxane, 1,2-dimethoxyethane, preferably in tetrahydrofuran or methyl-tetrahydrofuran, advantageously at a temperature between 25°C and -10°C, preferably at about 0°C.

The reaction leading from the activated compound (IIa) to the compound (III) provide for the sequential addition of N-hydroxy-imide, preferably N-hydroxy-phthalimide, an inorganic base selected from alkali carbonates, preferably potassium carbonate, and an organic amine, preferably dimethylaminopyridine. The reaction is carried out in suitable solvents, for example selected from tetrahydrofuran, methyltetrahydrofuran, dioxane, 1,2-dimethoxyethane, acetone, methyl ethyl ketone, methyl isobutyl ketone, acetonitrile, preferably in tetrahydrofuran or methyl-tetrahydrofuran, at a temperature preferably between 0°C and 80°C, preferably between 30°C and 60°C, advantageously at about 50°C.

In step (b), the compound of formula (III) is converted into the compound of formula (IV) by reaction with 3-ethyl-2-hydroxymethylacetophenone, without the need to purify the intermediate (IIIa) coming from the hydrolysis of the group X. Said hydrolysis reaction is carried out in the presence of an appropriate organic base, preferably selected from hydrazine or an aliphatic amine such as an alkylamine, advantageously n-butylamine in an suitable protic polar solvent, preferably selected from linear or branched C1-C4 alcohols, advantageously methanol.

The intermediate (IV) can then be obtained by adding 3-ethyl-2-hydroxymethylacetophenone to the intermediate (IIIa) in a protic polar solvent, preferably selected from linear or branched C1-C4 alcohols, in a mixture with water, advantageously using the methanol/water mixture, preferably in a ratio of about 1/1 (volume/volume).

Both reactions of step (b) are preferably carried out at a temperature between 15°C and 50°C, more preferably at about 25-35°C.

The compound (IV) isolated in step (b) in solid form, is used in step (c) to be transformed, through three reactions, into the compound (I), the key intermediate in the synthesis of Siponimod, again without the need to purify the intermediates.

The first reaction of step (c) provides for a protection of the hydroxyl function with the reagent R₄Cl, where R₄ is any of the protective groups known in organic chemistry, e.g. selected from those described in the literature in various texts, for example in "Greene's Protective Groups in Organic Chemistry", John Wiley & Sons, Ltd, Ed. 2007, preferably by using trimethylsilyl chloride (TMSCl) or tert-butyldimethylsilyl chloride (TBSCl), advantageously tert-butyldimethylsilyl chloride (TBSCl).

Said reaction is carried out in a suitable solvent, preferably in a polar aprotic solvent, advantageously selected from methylene chloride, chloroform, 1,2-dichloroethane, chlorobenzene, dichlorobenzene, preferably in methylene chloride. The reaction is preferably carried out in the presence of a base, for example an amine which captures the hydrochloric acid formed during the reaction. Said amine is preferably selected from triethylamine, pyridine and dimethylaminopyridine (DMAP) and is advantageously dimethylaminopyridine (DMAP).

The protective reaction is carried out at a temperature preferably between 25°C and -10°C, preferably at about 0°C.

The so-protected intermediate (IVa) can be transformed into the intermediate (IVb) without the need for purification by an addition reaction with a Grignard reagent, preferably selected from cyclohexylmagnesium chloride and cyclohexylmagnesium bromide, advantageously in the presence of zinc bromide or zinc chloride and Palladium metal complexes selected from Pd[P(But)₃]₂.

Said addition reaction is preferably carried out in ZnCl₂ and with Pd[P(But)₃]₂.

The solvent of the addition reaction is preferably selected from a high-boiling aprotic polar solvent advantageously selected from dimethylformamide (DMF) and N-methylpyrrolidone (NMP), preferably N-methylpyrrolidone (NMP).

The intermediate (IVb), without the need for purification, is subsequently oxidized to give the compound (I), after deprotection reaction to give the compound (IVc).

The deprotection reaction may be carried out in a suitable solvent, preferably in a solvent miscible with water, advantageously selected from tetrahydrofuran, dioxane and acetonitrile, preferably in tetrahydrofuran, and preferably in the presence of pyridinium p-toluenesulfonate (PTTS), at a temperature preferably between 15°C and 40°C, more preferably at about 25°C.

The oxidation reaction leading to the compound (I) may be carried out in a suitable solvent, preferably an apolar solvent such as hexane, heptane, petroleum ether, toluene, advantageously heptane in the presence of an oxidizing agent useful to convert the alcohol group to aldehyde.

Preferably, manganese dioxide and TEMPO (2,2,6,6-tetramethylpiperidin-1-yl)oxyl), preferably manganese dioxide, may be conveniently used.

The desired compound (I) can be isolated from the reaction environment according to conventional methods, for example by using a crystallization process, for example in a mixture of suitable solvents, for example selected from alcohols and water, advantageously isopropanol and water with an overall yield of 28.0%.

The compound of formula (I) can be transformed into Siponimod according to known techniques.

It is also disclosed the use of the compound of formula (I) obtained according to the process of the invention as an intermediate for the preparation of Siponimod.

Some intermediate compounds of the process of the invention are novel compounds and are a further object of the invention.

Therefore, object of the invention is also an intermediate compound selected from the compounds of formula (IV), (IVa), and (IVa') having the following formulas: 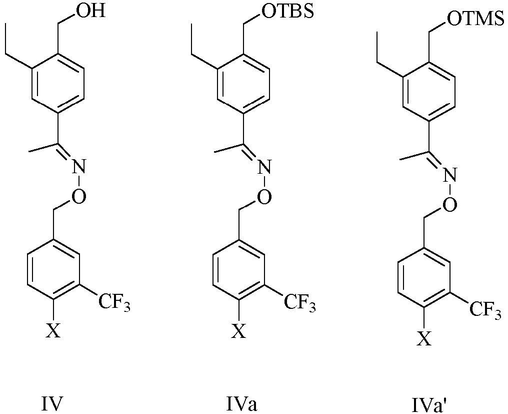 wherein X=Cl, Br.

A further object of the invention is the use of at least one of the compounds of formula (IIa), (IIa'), (III), (IV), (IVa), (IVa'), (IVb) and (IVb') having the following formulas: 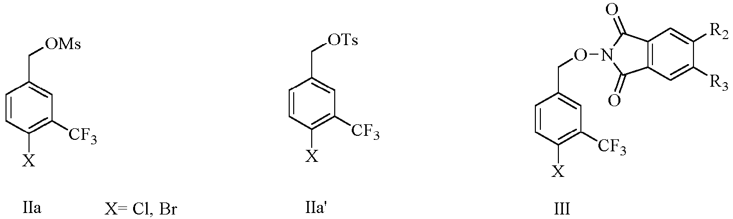 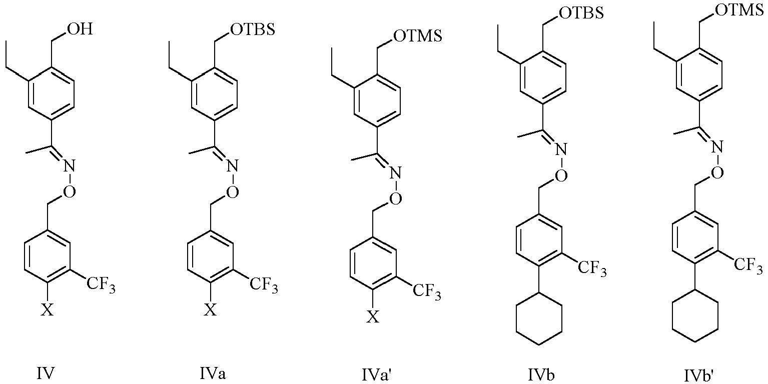 wherein R₂ and R₃, each independently, represent hydrogen, C1-C4-alkyl, halogen, nitro or R₂ and R₃ together form an aliphatic or aromatic, 5 to 7 membered ring, as intermediates for the preparation of Siponimod.

A further object of the invention is the use of the compounds of formula (IIa), (IIa'), (III), (IV), (IVa), (IVa'), (IVb) and (IVb') as described above as synthetic intermediates.

Preparation examples are provided in the Experimental Section of the present description for illustrative purposes only and in no way limiting.

### EXPERIMENTAL SECTION

### Abbreviations

THF tetrahydrofuran
Me-THF methyl tetrahydrofuran
NEt₃ triethylamine
MsCl mesylchloride
IPA isopropyl alcohol
DMAP dimethylaminopyridine
MTBE methyl tert-butyl ether
TBS-Cl tert-butyldimethylsilyl chloride
TMS-Cl trimethylsilyl chloride
NMP N-methylpyrrolidone
Pd(PBut₃)₂ tri-tert-butylphosphine palladium
AcOEt Ethyl acetate
PPTS pyridinium p-toluenesulfonate
TLC thin layer chromatography

### Analysis

NMR analysis was carried out with a Bruker 300 MHz AV device, Solvent: DMSO-d6.

The gas chromatography-mass spectrometry analysis was carried out with an Agilent 5977° GC-MS device, Column SPB530x0.25mmx0.25µm.

### Example 1 (step (a), synthesis of the compound (IIa))

In a reactor under a nitrogen atmosphere the following are added in this order at room temperature: 4-chloro-3-(trifluoromethyl)benzyl alcohol (25.0 g, 0.1187 moles), Me-THF and NEt₃ (0.1523 moles). The mixture is cooled to 0-5°C and a solution of MsCl (0.1306 moles) in Me-THF is added while maintaining the temperature below 10°C. The suspension is stirred at 0-5°C until complete conversion. Water is added and the phases are separated. The organic phase is washed in this order with a solution of 1.2 N HCl, water and a saturated aqueous solution of NaCl. The solvent is evaporated under vacuum at a temperature of ≤50°C. The oil obtained is subjected to the next step without further purification. 34.0 g of residue are obtained. A small sample is purified by column chromatography for the characterization:
¹H-NMR (300 MHz, DMSO): δ = 7.95 (1 H, s); 7.79 (2 H, br. s); 5.36 (2 H, s); 3.29 (3 H, s) ppm.
¹³C-NMR (75 MHz, DMSO): δ = 134.43; 134.20; 132.02; 131.12 (q, JC-F = 1.9 Hz); 127.84 (q, JC-F = 5.3 Hz); 126.80 (q, JC-F = 31.0 Hz); 122.67 (Q, JC-F = 273.1 Hz); 69.49; 37.00 ppm.

### Example 2 (step (a), synthesis of the compound (III))

In a reactor under nitrogen atmosphere, N-hydroxy-phthalimide (0.04780 moles), Me-THF (270 mL), the compound obtained in Example 1 (12.0 g, 0.04157 moles), K₂CO₃ (0.1954 moles) and DMAP (0.0021 moles) are added in this order at room temperature. The suspension is heated to 50±5°C until complete conversion. The reaction is cooled to <30°C and water is added. The organic phase is separated and the same is washed with water. The solvent is concentrated under vacuum at a temperature of <50°C. The residue is dissolved under reflux in 4 volumes of Me-THF and the solution is cooled to room temperature. The resulting suspension is stirred at room temperature overnight and subsequently cooled to 0-5°C for 2-3 hours. The resulting solid is filtered, washed with cold Me-THF and dried at 40°C to a constant weight. 10.4 g of solid obtained, molar yield 70.3%
¹H-NMR (300 MHz, DMSO): δ = 8.07 (1 H, d, *J* = 1.9 Hz); 7.87-7.83 (5 H, m); 7.76 (1 H, d, *J* = 8.2 Hz); 5.28 (2H, s) ppm.
¹³C-NMR (75 MHz, DMSO): δ = 163.05; 135.02; 134.82; 131.71; 131.15 (q, *J*_{C-F} = 1.8 Hz); 128.79 (q, *J*_{C-F} = 5.3 Hz); 128.47; 126.61 (q, *J*_{C-F} = 30.9 Hz); 123.30; 122.73 (q, *J*_{C-F} = 273.1 Hz); 77.56 ppm

### Example 3 (step (b), synthesis of the compound (IIIa))

In a reactor under nitrogen atmosphere the compound of Example 2 (0.0843 moles) and MeOH are added in this order at room temperature. Next, N-butylamine (0.09695 moles) is added dropwise and the reaction mixture is heated to 30±3°C until complete conversion. The solution is concentrated under vacuum at a temperature of ≤45°C. The residue obtained is diluted with MTBE and water. HCl (0.1265 moles) is added dropwise while maintaining the temperature at <30°C. The phases are separated and the aqueous phase is washed twice with MTBE. K₂CO₃ (0.1265 moles) is added in portions to the aqueous phase and extracted with MTBE. The organic phase is separated and is then washed with water. The solvent is evaporated under vacuum at a temperature of ≤45°C. The residue is subjected to the next step without further purification.

### Example 4 (step (b), synthesis of the compound (IV))

In a reactor under nitrogen atmosphere the residue obtained in Example 3 (9.4 g, 0.04167 moles), MeOH (38 mL) and water (3.8 mL) are added in this order at room temperature. Next, a solution of 4M HCl (0.0063 moles) is added and a solution of 1-(3-ethyl-4-hydroxymethyl-phenyl)-ethanone (0.03815 moles) dissolved in MeOH (16 mL) is added dropwise. The resulting suspension is stirred at room temperature for 20-24 h. The resulting solid is filtered and washed with an 8:2 v/v MeOH/H₂O mixture. The product is dried at 40°C to a constant weight. 12.2 g of solid obtained, molar yield 83.0%.

¹H-NMR (300 MHz, DMSO): δ = 7.88 (1H, s); 7.72 (2H, m); 7.45-7.38 (3 H, m); 5.27 (2 H, s); 5.14 (1H, t, *J* = 5.4 Hz, D₂O exchange); 4.54 (2H, d, *J* = 5.4 Hz); 2.61 (2 H, q, *J* = 7.5 Hz); 2.23 (3 H, s); 1.14 (3 H, t, *J* = 7.5 Hz) ppm.

¹³C-NMR (75 MHz, DMSO): δ = 155.40; 141.10; 140.98; 138.42; 134.19, 133.54; 131.66; 129.83 (q, *J*_{C-F} = 1.9 Hz); 127.19 (q, *J*_{C-F} = 5.3 Hz); 127.07; 126.46 (q, *J*_{C-F} = 30.7 Hz); 125.33; 123.30; 122.84 (q, *J*_{C-F} = 273.0 Hz); 73.48; 60.24; 24.48; 14.84; 12.63 ppm.

### Example 5 (step (c), synthesis of the compound (IVa))

In a reactor under nitrogen atmosphere the compound obtained in Example 4 (17.5 g, 0.04536 moles), CH₂Cl₂ and imidazole (0.05262 moles) are added in this order at room temperature. The solution is cooled to 0-5°C and TBS-Cl (0.05080 moles) is added while maintaining the temperature of <10°C. A catalytic amount of DMAP is added and allowed to raise at room temperature until complete conversion. Water is added and the phases are separated. The organic phase is washed with aqueous formic acid and twice with water. The solvent is distilled under vacuum at a temperature of <40°C and toluene is added. The toluene is removed under vacuum at a temperature of <50°C. 24.0 g of residue obtained. The residue is subjected to the next step without further purification.

### Example 6 (step (c), synthesis of the compound (IVb))

In a reactor under nitrogen atmosphere, NMP (44 mL) and anhydrous ZnCl₂ (0.09979 moles) are added in this order at room temperature. The suspension is heated to 105-115°C for a few minutes and the solution is cooled to room temperature. A 2M solution of cyclohexylmagnesium chloride in Et₂O (0.09072 moles) is added dropwise while maintaining the temperature <60°C. The reaction is cooled to room temperature until a non-stirrable solid is given. It is heated to 80-90°C for 1 h to remove excess Et₂O and cooled to room temperature. The crude compound obtained in Example 5 (0.04536 moles), NMP (6 mL), and Pd(PBut₃)₂ (0.0032 mL) are added and the reaction mixture is heated to 135-145°C for 1-1.5 hours. It is cooled to 80-90°C and water and cyclohexane are added. AcOEt is added while maintaining the temperature below 40°C. The mixture is filtered onto cellulose and the panel is washed with 4:1 v/v cyclohexane/AcOEt. The phases are separated and the aqueous layer is extracted with 4:1 v/v cyclohexane/AcOEt. The combined organic phases are washed with water and the solvent is distilled under vacuum at a temperature of <45°C. 18.4 g of residue obtained. The residue is dissolved in a 4.5:1 v/v cyclohexane/AcOEt mixture and filtered over a small amount of silica gel. The solvent is removed under vacuum at a temperature of <45°C. 18.0 g of residue obtained. The residue is subjected to the next step without further purification.

### Example 7 (step (c), synthesis of the compound (IVc))

In a reactor under nitrogen atmosphere the crude compound of Example 6 (0.04536 moles), a 10:1 v/v THF/water mixture and a catalytic amount of PPTS are added in this order at room temperature. The solution is stirred at room temperature until complete conversion. The solvent is distilled under vacuum at a temperature of <50°C. Toluene and water are added to the residue. After the separation, the organic phase is washed with water. The solution is distilled under vacuum at a temperature of <50°C. 15.0 g of residue obtained. The residue is subjected to the next step without further purification.

A portion of the residue is purified for the analysis.

¹H-NMR (300 MHz, DMSO): δ = 7.69-7.63 (3 H, m); 7.45-7.37 (3 H, m); 5.22 (2 H, s); 5.13 (1 H, t, *J* = 5.4 Hz, D₂O exchange); 4.53 (2 H, d, *J* = 5.4 Hz); 2.81 (1 H, m); 2.61 (2 H, q, *J* = 7.5 Hz); 2.21 (3 H, s); 1.82-1.29 (10 H, m); 1.15 (3 H, t, *J* = 7.5 Hz) ppm.

¹³C-NMR (75 MHz, DMSO): δ = 154.92; 145.65; 141.00; 140.97; 136.31; 134.30; 132.28; 128.54; 127.06; 126.05 (q, *J*_{C-F} = 28.3 Hz); 125.31; 125.02 (q, *J*_{C-F} = 5.8 Hz); 124.65 (q, *J*_{C-F} = 274.0 Hz); 123.27; 74.19; 60.23; 39.70; 33.84; 26.41; 25.38; 24.47; 14.86; 12.61 ppm.

GC-MS: 433.3.

### Example 8 (step (c), synthesis of the compound (I))

In a reactor under nitrogen atmosphere the crude compound of Example 7 (0.04536 moles), n-heptane and MnO₂ (0.3016 moles) are added in this order at room temperature. The suspension is heated to 45-55°C until complete conversion. It is diluted with toluene and then cooled to room temperature. The solid is filtered and washed with toluene. The solution is concentrated under vacuum at a temperature of <50°C. Residue obtained = 13.0 g. The residue is dissolved in a 30:1 v/v PAH/H₂O mixture (18 mL). Then the solution is cooled to room temperature for 20-24 hours and then to 0-5°C for 1-2 hours. The thus obtained suspension is filtered and the solid is washed with a 30:1 v/v IPA/H₂O mixture (2x10 mL). The solid is dried at 40°C to a constant weight. 9.4 g of solid obtained, molar yield = 48.0% over four steps (isolated compounds).

¹H-NMR (300 MHz, DMSO): δ = 10.25 (1 H, s); 7.85 (1 H, d, *J* = 8.1 Hz); 7.70-7.61 (5 H, m); 5.28 (2 H, s); 3.05 (2 H, q, *J* = 7.5 Hz); 2.81 (1 H, m); 2.25 (3 H, s); 1.82-1.29 (10 H, m); 1.18 (3 H, t, *J* = 7.5 Hz) ppm.

¹³C-NMR (75 MHz, DMSO): δ = 192.58; 154.34; 146.56; 145.80; 140.53; 135.95; 133.53; 132.34; 131.46; 128.59; 127.62; 126.09 (q, *J*_{C-F} = 28.9 Hz); 125.09 (q, *J*_{C-F} = 6.0 Hz); 124.61 (q, *J*_{C-F} = 274.2 Hz); 123.95; 74.63; 39.70; 33.82; 26.40; 25.37; 24.97; 16.35; 12.47 ppm.

GC-MS: 431.2

### Example 9 (step (c), synthesis of the compound (IVa'))

In a reactor under nitrogen atmosphere the compound obtained in Example 4 (11.0 g, 0.02856 moles), CH₂Cl₂ (10 volumes) and imidazole (0.04284 moles) are added in sequence at room temperature. The solution is cooled to 0-5°C and TMS-Cl (0.04141 moles) is added dropwise while maintaining the temperature below 10°C. Then a catalytic amount of DMAP is added and the reaction is maintained at room temperature until complete conversion. The reaction is cooled to 0-5°C for 30'-1 h, the solid obtained is filtered and the panel is washed with CH₂Cl₂. The filtrate is concentrated under vacuum at 40°C until an oily residue of constant weight is obtained.

The residue is subjected to the next step without further purification.

GC-MS: 457.2

### Example 10 (step (c), synthesis of the compound (IVb'))

In a reactor under nitrogen atmosphere, NMP (27 mL) and anhydrous ZnCl₂ (0,0628 moles) are added in sequence at room temperature. The suspension is heated to 100-110°C for a few minutes, then it is cooled to room temperature. A 2M solution of cyclohexylmagnesium chloride (0.05712 moles) is added dropwise while maintaining the temperature below 60°C. The reaction is cooled to room temperature until a non-stirrable solid is given. It is heated to 80-90°C for 1 h to remove excess Et₂O and cooled to room temperature. The crude compound obtained in Example 9 (0.02856 moles), NMP (3 mL) and Pd(PBut₃)₂ (0.002 moles) are added and is heated to 135-145°C for 1-1.5 hours. The reaction is cooled to T<100°C and NMP (15 mL) and toluene (80 mL) are added. It is cooled to 60°C and water (80 mL) and AcOEt (40 mL) are added. The mixture is filtered onto cellulose and the panel is washed with 2:1 v/v Toluene/AcOEt (2x30 mL). The organic phase is separated and the aqueous phase is washed with 2:1 v/v Toluene/AcOEt (60 mL). The combined organic phases are washed with a saturated NaCl solution (100 mL). The organic solvent is removed under vacuum at a temperature below 50°C. The residue is subjected to the next step without further purification.

GC-MS: 505.3

### Example 11 (step (c), synthesis of the compound (IVc))

In a reactor under nitrogen atmosphere the crude compound obtained in Example 10 (0,02856 moles), a 10:1 v/v CH₃CN/water mixture and NH₄Cl (0,04284 moles) are added in sequence at room temperature. The suspension is heated to 45-55°C until complete conversion. The solvent is removed under vacuum at a temperature below 50°C. The residue is dissolved in a mixture consisting of 1:2 v/v toluene/EtOAc and water. The organic phase is separated and the solvent is removed at a temperature below 50°C. The residue may be subjected to the next step according to Example 8 without any purification. A sample of compound (IVc) was purified for the analytical check.

¹H-NMR (300 MHz, DMSO): δ = 7.69-7.63 (3 H, m); 7.45-7.37 (3 H, m); 5.22 (2 H, s); 5.13 (1 H, t, *J* = 5.4 Hz, D2O exchange); 4.53 (2 H, d, *J* = 5.4 Hz); 2.81 (1 H, m); 2.61 (2 H, q, *J* = 7.5 Hz); 2.21 (3 H, s); 1.82-1.29 (10 H, m); 1.15 (3 H, t, *J* = 7.5 Hz) ppm.

¹³C-NMR (75 MHz, DMSO): δ = 154.92; 145.65; 141.00; 140.97; 136.31; 134.30; 132.28; 128.54; 127.06; 126.05 (q, *J*_{C-F} = 28.3 Hz); 125.31; 125.02 (q, *J*_{C-F} = 5.8 Hz); 124.65 (q, *J*_{C-F} = 274.0 Hz); 123.27; 74.19; 60.23; 39.70; 33.84; 26.41; 25.38; 24.47; 14.86; 12.61 ppm.

GC-MS: 433.3.

### Comparative Example

### Attempt to introduce the cyclohexyl group on the compound of formula (III)

In a reactor under nitrogen atmosphere, NMP (27 mL) and anhydrous ZnCl₂ (0,06182 moles) are added in this order at room temperature. The suspension is heated to 100-110°C and then cooled to room temperature. A 2M solution of cyclohexylmagnesium chloride in Et₂O (0.0562 moles) is added dropwise while maintaining the temperature below 60°C. The reaction is cooled to room temperature until a non-stirrable solid is obtained. It is heated to 80-85°C for 0.5-1 h to remove the excess solvent, then it is cooled to room temperature. The compound of the Example 2 (0.0281 moles) and Pd(PBut₃)₂ (0,0014 moles) are added and the reaction is heated to 130-140°C for 1-1.5 hours. The reaction evaluated in TLC (7:3 v/v n-heptane/AcOEt) shows that the starting reagent disappeared. It is cooled to room temperature and a mixture of 3:2 v/v n-heptane /AcOEt is added. Next, a solution of 1,2N HCl in water is added and heated to 50-60°C. It is cooled to room temperature and the phases are separated. The aqueous phase is extracted with 3:2 v/v n-heptane/AcOEt and the combined organic phases are washed with water. The organic phase is filtered onto cellulose and washed with AcOEt. The solution is concentrated under vacuum at 40-50°C. The obtained oil is dissolved in THF, hydrazine monohydrate is added and the course of the reaction is followed by TLC. The desired product was not obtained.

## Claims

1. A process for the preparation of the compound of formula (I) 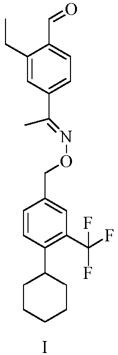 comprising the following steps:
a) converting the compound of formula (II) into the compound of formula (III), passing through the formation of intermediate (IIa), 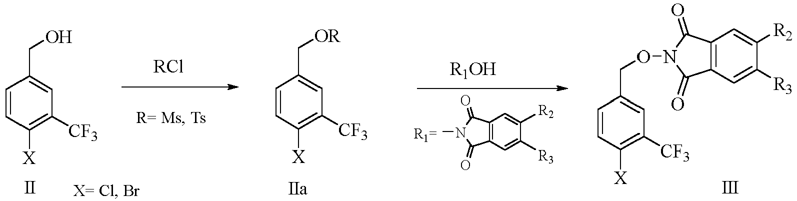 wherein R is selected from mesyl and tosyl and R₁ is a group of formula 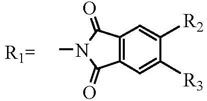
wherein R₂ and R₃, each independently, represent hydrogen, C1-C4-alkyl, halogen, nitro or R₂ and R₃ together form an aliphatic or aromatic, 5 to 7 membered ring,
in suitable solvents;
b) converting the thus obtained compound of formula (III) into the compound of formula (IV) by the hydrolysis of the R₁ group and the subsequent reaction with 3-ethyl-2-hydroxymethylacetophenone, passing through the formation of intermediate (IIIa) according to the following scheme, in suitable solvents; 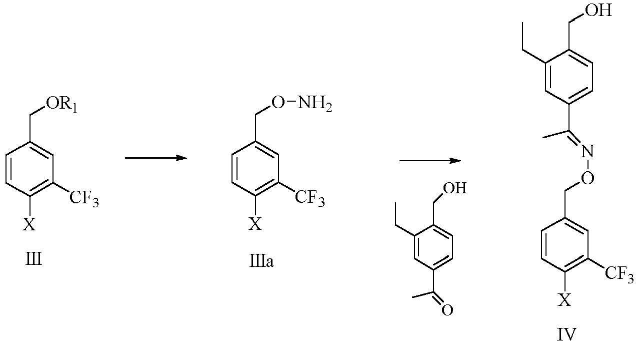
c) converting the compound of formula (IV) into the compound of formula (I), in the presence of metal catalysts and an oxidizing agent, upon the protection of the hydroxy group, introduction of the cyclohexyl group and deprotection of the hydroxy group, passing through the formation of the compounds (IVa), (IVb), (IVc) according to the following scheme 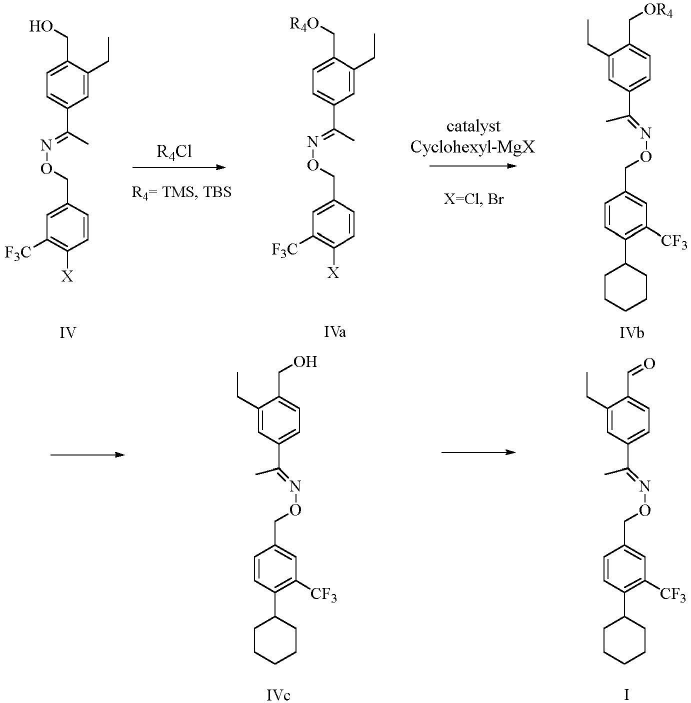
wherein R₄ is selected from a protecting group of the hydroxy function, in suitable solvents;
wherein some or all of the compounds (IIa), (IIIa), (IVa), (IVb) and (IVc) are not isolated from the reaction crude and/or purified.

2. The process according to claim 1, **characterized in that** in step (a) mesyl chloride is used, R₂ and R₃ are hydrogen, and said solvents are selected from tetrahydrofuran, methyl-tetrahydrofuran, dioxane, 1,2-dimethoxyethane and mixtures thereof, preferably in tetrahydrofuran or methyl-tetrahydrofuran.

3. The process according to claim 1 or 2, **characterized in that** in step (b) the hydrolysis is carried out in the presence of a suitable organic base, preferably selected from hydrazine or an aliphatic amine such as an alkylamine, more preferably n-butylamine in a protic polar solvent, preferably selected from straight or branched C1-C4 alcohols and mixtures thereof, and the transformation into the compound of formula (IV) is carried out in a protic polar solvent, preferably selected from straight or branched C1-C4 alcohols and mixtures thereof, in mixture with water, advantageously by using the methanol/water mixture, preferably in a ratio of about 1/1 (volume/volume).

4. The process according to any one of claims 1 to 3, **characterized in that** in step (c), R₄Cl is selected from trimethylsilyl chloride (TMSCl) or tert-butyldimethylsilyl chloride (TBSCl), advantageously tert-butyldimethylsilyl chloride (TBSCl), the solvent for the synthesis of the compound of formula (IVa) is an aprotic polar solvent, preferably selected from methylene chloride, chloroform, 1,2-dichloroethane, chlorobenzene, dichlorobenzene and mixtures thereof, more preferably in methylene chloride, and the reaction is carried out in the presence of an amine, preferably selected from triethylamine, pyridine and dimethylaminopyridine (DMAP), more preferably dimethylaminopyridine (DMAP).

5. The process according to any one of claims 1 to 4, **characterized in that** the intermediate (IVa) is converted into the intermediate (IVb) by reacting with cyclohexylmagnesium chloride or cyclohexylmagnesium bromide, in the presence of zinc bromide or zinc chloride and of palladium metal complexes selected from Pd[P(But)₃]₂, in a high-boiling aprotic polar solvent, advantageously selected from dimethylformamide (DMF), N-methylpyrrolidone (NMP) and mixtures thereof, preferably N-methylpyrrolidone (NMP).

6. The process according to any one of claims 1 to 5, **characterized in that** the intermediate (IVa) is converted into the intermediate (IVb) by a deprotection reaction carried out in a suitable solvent, preferably in a water-miscible solvent, more preferably selected from tetrahydrofuran, dioxane, acetonitrile and mixtures thereof, preferably in tetrahydrofuran, in the presence of pyridinium p-toluenesulfonate (PTTS).

7. The process according to any one of claims 1 to 6, **characterized in that** the oxidation reaction of step (c), which leads to compound (I), is carried out in a solvent selected from an apolar solvent, preferably selected from hexane, heptane, petroleum ether, toluene and mixtures thereof, more preferably heptane, in the presence of an oxidizing agent selected from manganese dioxide and TEMPO (2,2,6,6-tetramethylpiperidin-1-yl)oxyl), preferably manganese dioxide.

8. The process according to any one of claims 1 to 7, **characterized in that** none of the compounds (IIa), (IIIa), (IVa), (IVb) and (IVc) is isolated from the reaction crude and/or purified.

9. A compound selected from the compounds having the following formulas: 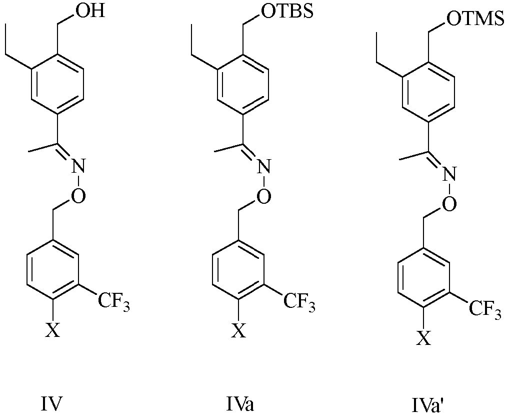 wherein X=Cl, Br.

10. Use of at least one of the compounds having the following formulas: 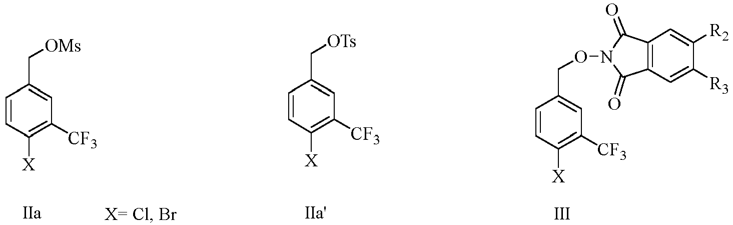 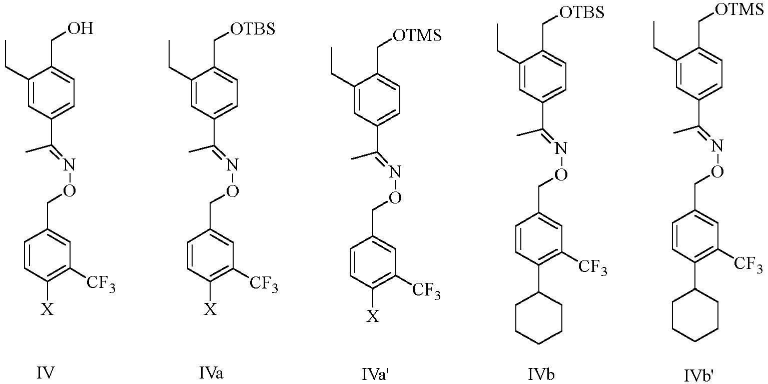 wherein R₂ and R₃, each independently, represent hydrogen, C1-C4-alkyl, halogen, nitro or R₂ and R₃ together form an aliphatic or aromatic, 5 to 7 membered ring, in the preparation of Siponimod.

## Patentansprüche

1. Verfahren zur Herstellung der Verbindung der Formel (I) 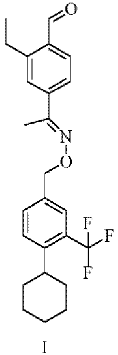 umfassend folgende Schritte:
a) Umwandeln der Verbindung der Formel (II) in die Verbindung der Formel (III) über die Bildung des Zwischenprodukts (IIa), 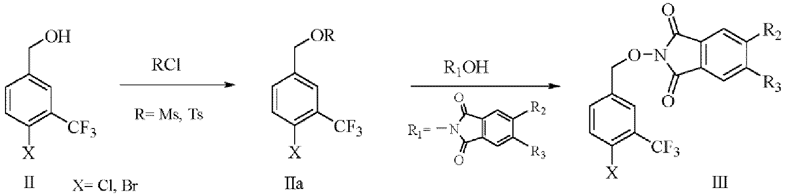 wobei R ausgewählt ist aus Mesyl und Tosyl und R₁ eine Gruppe der Formel 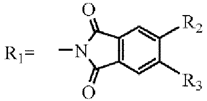
ist, wobei R₂ und R₃ jeweils unabhängig Wasserstoff, C₁-C₄-Alkyl, Halogen, Nitro darstellen oder R₂ und R₃ zusammen einen alphatischen oder aromatischen, 5- bis 7-gliedrigen Ring bilden,
in geeigneten Lösungsmitteln;
b) Umwandeln der so gewonnenen Verbindung der Formel (III) in die Verbindung der Formel (IV) durch Hydrolyse der R₁-Gruppe und anschließende Reaktion mit 3-Ethyl-2-Hydroxymethylacetophenon über die Bildung des Zwischenprodukts (IIIa) nach dem folgenden Schema in geeigneten Lösungsmitteln; 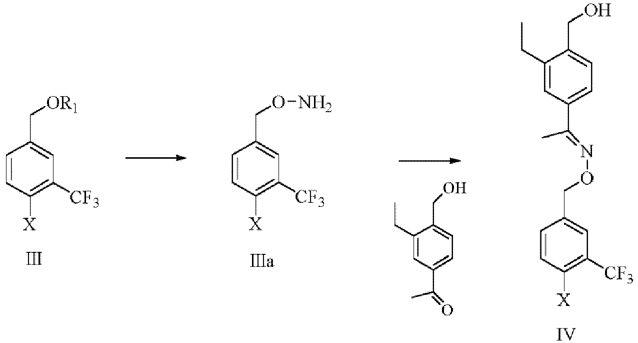
c) Umwandeln der Verbindung der Formel (IV) in die Verbindung der Formel (I) in Gegenwart von Metallkatalysatoren und einem Oxidationsmittel, nach dem Schutz der Hydroxygruppe Einführen der Cyclohexylgruppe und Entschützen der Hydroxygruppe über die Bildung der Verbindungen (IVa), (IVb), (IVc) nach dem folgenden Schema 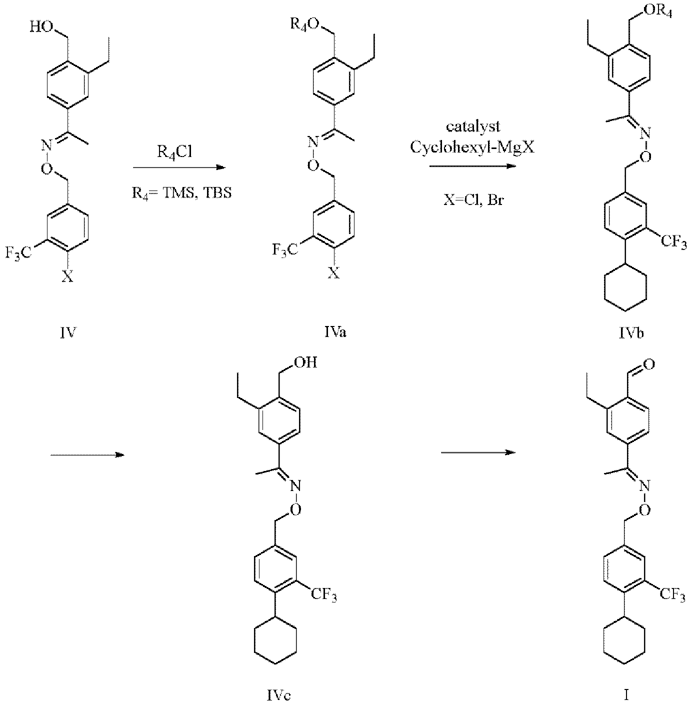 ***Katalysator** Cyclohexyl-MgX*
wobei R₄ ausgewählt ist aus einer Schutzgruppe der Hydroxyfunktion, in geeigneten Lösungsmitteln;
wobei einige oder alle der Verbindungen (Ila), (IIIa), (IVa), (IVb) und (IVc) nicht von dem Reaktionsrohstoff isoliert und/oder gereinigt werden.

2. Verfahren nach Anspruch 1, **dadurch gekennzeichnet, dass** in Schritt (a) Mesylchlorid verwendet wird, R₂ und R₃ Wasserstoff sind und die Lösungsmittel ausgewählt sind aus Tetrahydrofuran, Methyl-Tetrahydrofuran, Dioxan, 1,2-Dimethoxyethan und deren Gemischen, vorzugsweise in Tetrahydrofuran oder Methyl-Tetrahydrofuran.

3. Verfahren nach Anspruch 1 oder 2, **dadurch gekennzeichnet, dass** in Schritt (b) die Hydrolyse in Gegenwart einer geeigneten organischen Base, vorzugsweise ausgewählt aus Hydrazin oder einem aliphatischen Amin wie einem Alkylamin, insbesondere vorzugsweise n-Butylamin in einem polaren protischen Lösungsmittel, vorzugsweise ausgewählt aus geradkettigen oder verzweigten C₁-C₄-Alkoholen und deren Gemischen, durchgeführt wird und das Umwandeln in die Verbindung der Formel (IV) in einem polaren protischen Lösungsmittel durchgeführt wird, vorzugsweise ausgewählt aus geradkettigen oder verzweigten C₁-C₄-Alkoholen und deren Gemischen, in einem Gemisch mit Wasser, vorteilhafterweise unter Verwendung des Methanol/Wasser-Gemischs, vorzugsweise in einem Verhältnis von etwa 1/1 (Volumen/Volumen).

4. Verfahren nach einem der Ansprüche 1 bis 3, **dadurch gekennzeichnet, dass** in Schritt (c) R₄Cl ausgewählt ist aus Trimethylsilylchlorid (TMSCI) oder tert-Butyldimethylsilylchlorid (TBSCl), vorzugsweise tert-Butyldimethylsilylchlorid (TBSCl), das Lösungsmittel für die Synthese der Verbindung der Formel (IVa) ein polares aprotisches Lösungsmittel ist, vorzugsweise ausgewählt aus Methylenchlorid, Chloroform, 1,2-Dichlorethan, Chlorbenzol, Dichlorbenzol und deren Gemischen, insbesondere vorzugsweise in Methylenchlorid, und die Reaktion in Gegenwart eines Amins durchgeführt wird, vorzugsweise ausgewählt aus Triethylamin, Pyridin und Dimethylaminopyridin (DMAP), insbesondere vorzugsweise Dimethylaminopyridin (DMAP).

5. Verfahren nach einem der Ansprüche 1 bis 4, **dadurch gekennzeichnet, dass** das Zwischenprodukt (IVa) durch Reagieren mit Cyclohexylmagnesiumchlorid oder Cyclohexylmagnesiumbromid in Gegenwart von Zinkbromid oder Zinkchlorid und von Palladiummetallkomplexen, ausgewählt aus Pd[P(But)₃]₂, in einem hochsiedenden, polaren aprotischen Lösungsmittel, vorteilhafterweise ausgewählt aus Dimethylformamid (DMF), N-Methylpyrrolidon (NMP) und deren Gemischen, vorzugsweise N-Methylpyrrolidon (NMP), in das Zwischenprodukt (IVb) umgewandelt wird.

6. Verfahren nach einem der Ansprüche 1 bis 5, **dadurch gekennzeichnet, dass** das Zwischenprodukt (IVa) durch eine Entschützungsreaktion, die in einem geeigneten Lösungsmittel, vorzugsweise in einem mit Wasser mischbaren Lösungsmittel, insbesondere vorzugsweise ausgewählt aus Tetrahydrofuran, Dioxan, Acetonitril und deren Gemischen, vorzugsweise in Tetrahydrofuran, in Gegenwart von Pyridinium-p-toluolsulfonat (PTTS) durchgeführt wird, in das Zwischenprodukt (IVb) umgewandelt wird.

7. Verfahren nach einem der Ansprüche 1 bis 6, **dadurch gekennzeichnet, dass** die Oxidationsreaktion von Schritt (c), die zu der Verbindung (I) führt, in einem Lösungsmittel durchgeführt wird, das ausgewählt ist aus einem apolaren Lösungsmittel, vorzugsweise ausgewählt aus Hexan, Heptan, Petrolether, Toluol und deren Gemischen, insbesondere vorzugsweise Heptan, in Gegenwart eines Oxidationsmittels, ausgewählt aus Mangandioxid und TEMPO (2,2,6,6-Tetramethylpiperidin-1-yl)oxyl), vorzugsweise Mangandioxid.

8. Verfahren nach einem der Ansprüche 1 bis 7, **dadurch gekennzeichnet, dass** keine der Verbindungen (Ila), (IIIa), (IVa), (IVb) und (IVc) von dem Reaktionsrohstoff isoliert und/oder gereinigt wird.

9. Verbindung, ausgewählt aus den Verbindungen, aufweisend die folgenden Formeln: 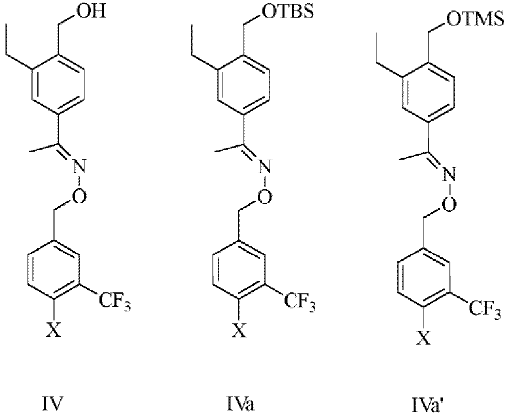 wobei X = C1, Br.

10. Verwendung von mindestens einer der Verbindungen mit den folgenden Formeln: 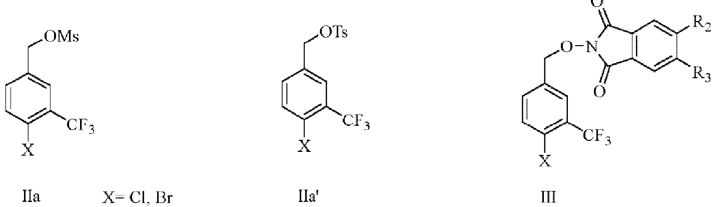 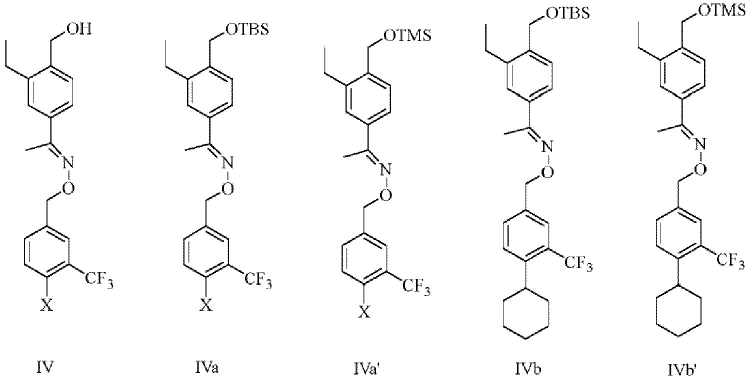 wobei R₂ und R₃ jeweils unabhängig Wasserstoff, C₁-C₄-Alkyl, Halogen, Nitro darstellen oder R₂ und R₃ zusammen einen alphatischen oder aromatischen, 5- bis 7-gliedrigen Ring bilden, bei der Herstellung von Siponimod.

## Revendications

1. Procédé de préparation du composé de formule (I) 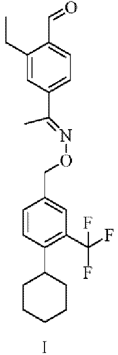 qui comprend les étapes suivantes:
a) conversion du composé de formule (II) en composé de formule (III), en passant par la formation de l'intermédiaire (IIa), 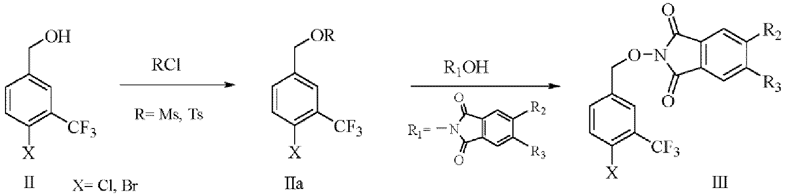 dans lequel R est choisi parmi les mésyles et les tosyles et R₁ est un groupe de formule 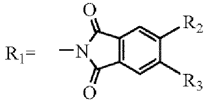
dans lequel R₂ et R₃, chacun indépendamment, représentent l'hydrogène, l'alkyle en C₁-C₄, l'halogène, le nitro ou R₂ et R₃ forment ensemble un anneau aliphatique ou aromatique de 5 à 7 membres,
dans des solvants appropriés;
b) conversion du composé de formule (III) ainsi obtenu en composé de formule (IV) par hydrolyse du groupe R₁ et réaction ultérieure avec la 3-éthyl-2-hydroxyméthylacétophénone, en passant par la formation de l'intermédiaire (IIIa) selon le schéma suivant, dans des solvants appropriés; 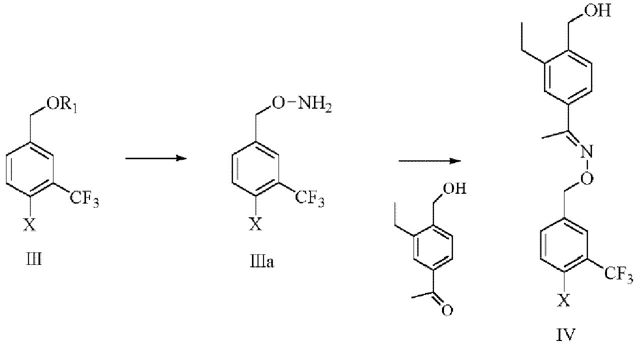
c) conversion du composé de formule (IV) en composé de formule (I), en présence de catalyseurs métalliques et d'un agent oxydant, après protection du groupe hydroxy, introduction du groupe cyclohexyle et déprotection du groupe hydroxy, en passant par la formation des composés (IVa), (IVb), (IVc) selon le schéma suivant 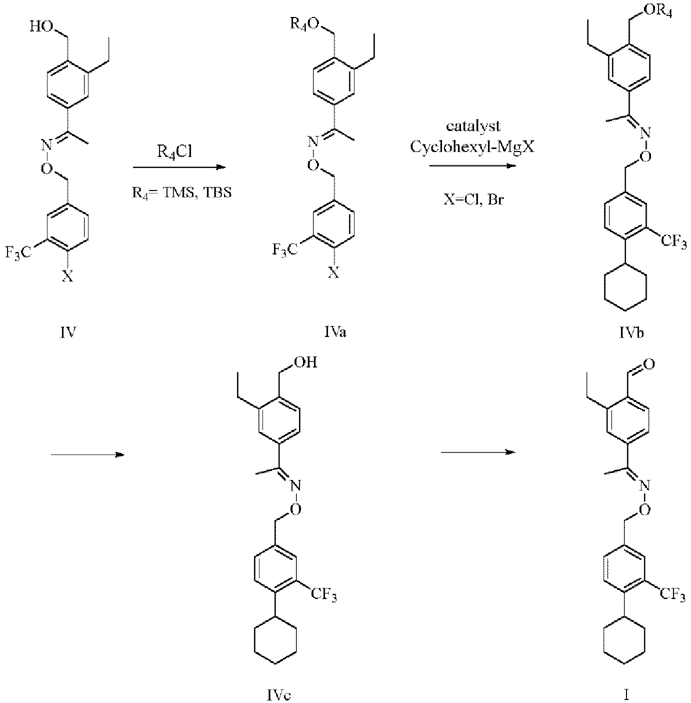 *catalyseur*
*Cyclohexyl-MgX*
dans lequel R₄ est choisi parmi un groupe de protection de la fonction hydroxy, dans des solvants appropriés;
dans lequel tout ou une partie des composés (Ila), (IIIa), (IVa), (IVb) et (IVc) ne sont pas isolés du brut de réaction et/ou purifiés.

2. Procédé selon la revendication 1, **caractérisé en ce qu'**à l'étape (a) on utilise du chlorure de mésyle, R₂ et R₃ sont de l'hydrogène, et lesdits solvants sont choisis parmi le tétrahydrofurane, le méthyl-tétrahydrofurane, le dioxane, le 1,2-diméthoxyéthane et leurs mélanges, de préférence dans le tétrahydrofurane ou le méthyl-tétrahydrofurane.

3. Procédé selon la revendication 1 ou 2, **caractérisé en ce qu'**à l'étape (b) l'hydrolyse est réalisée en présence d'une base organique appropriée, de préférence choisie parmi l'hydrazine ou une amine aliphatique telle qu'une alkylamine, plus préférentiellement la n-butylamine dans un solvant polaire protique, de préférence choisi parmi les alcools en C₁-C₄, linéaires ou ramifiés, et leurs mélanges, et la transformation en composé de formule (IV) est effectuée dans un solvant polaire protique, de préférence choisi parmi les alcools en C₁-C₄ linéaires ou ramifiés et leurs mélanges, en mélange avec de l'eau, avantageusement en utilisant le mélange méthanol/eau, de préférence dans un rapport d'environ 1/1 (volume/volume).

4. Procédé selon l'une quelconque des revendications 1 à 3, **caractérisé en ce qu'**à l'étape (c), R₄Cl est choisi parmi le chlorure de triméthylsilyle (TMSCI) ou le chlorure de tert-butyldiméthylsilyle (TBSCI), avantageusement le chlorure de tert-butyldiméthylsilyle (TBSCI), le solvant pour la synthèse du composé de formule (IVa) est un solvant polaire aprotique, de préférence choisi parmi le chlorure de méthylène, le chloroforme, le 1,2-dichloroéthane, le chlorobenzène, le dichlorobenzène et leurs mélanges, de préférence dans le chlorure de méthylène, et la réaction est effectuée en présence d'une amine, de préférence choisie parmi la triéthylamine, la pyridine et la diméthylaminopyridine (DMAP), de préférence la diméthylaminopyridine (DMAP).

5. Procédé selon l'une quelconque des revendications 1 à 4, **caractérisé en ce que** l'intermédiaire (IVa) est converti en l'intermédiaire (IVb) par réaction avec le chlorure de cyclohexylmagnésium ou le bromure de cyclohexylmagnésium, en présence de bromure de zinc ou de chlorure de zinc et de complexes métalliques de palladium choisis parmi Pd[P(But)₃]₂, dans un solvant polaire aprotique à point d'ébullition élevé, avantageusement choisi parmi le diméthylformamide (DMF), la N-méthylpyrrolidone (NMP) et leurs mélanges, de préférence la N-méthylpyrrolidone (NMP).

6. Procédé selon l'une quelconque des revendications 1 à 5, **caractérisé en ce que** l'intermédiaire (IVa) est converti en l'intermédiaire (IVb) par une réaction de déprotection effectuée dans un solvant approprié, de préférence dans un solvant miscible à l'eau, plus préférentiellement choisi parmi le tétrahydrofurane, le dioxane, l'acétonitrile et leurs mélanges, de préférence dans le tétrahydrofurane, en présence de p-toluènesulfonate de pyridinium (PTTS).

7. Procédé selon l'une quelconque des revendications 1 à 6, **caractérisé en ce que** la réaction d'oxydation de l'étape (c), qui conduit au composé (I), est effectuée dans un solvant choisi parmi un solvant apolaire, de préférence choisi parmi l'hexane, l'heptane, l'éther de pétrole, le toluène et leurs mélanges, plus préférentiellement l'heptane, en présence d'un agent oxydant choisi parmi le dioxyde de manganèse et le TEMPO (2,2,6,6-tetraméthylpipéridine-1-yl)oxyl), de préférence le dioxyde de manganèse.

8. Procédé selon l'une quelconque des revendications 1 à 7, **caractérisé en ce qu'**aucun des composés (IIa), (IIIa), (IVa), (IVb) et (IVc) n'est isolé du brut de réaction et/ou purifié.

9. Composé choisi parmi les composés ayant les formules suivantes: 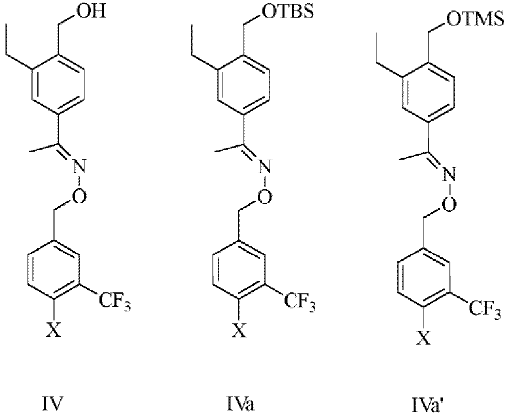 dans lequel X=C1, Br.

10. Utilisation d'au moins un des composés ayant les formules suivantes: 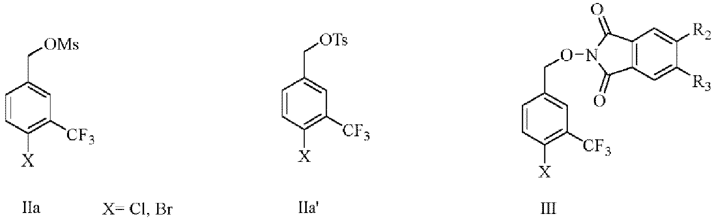 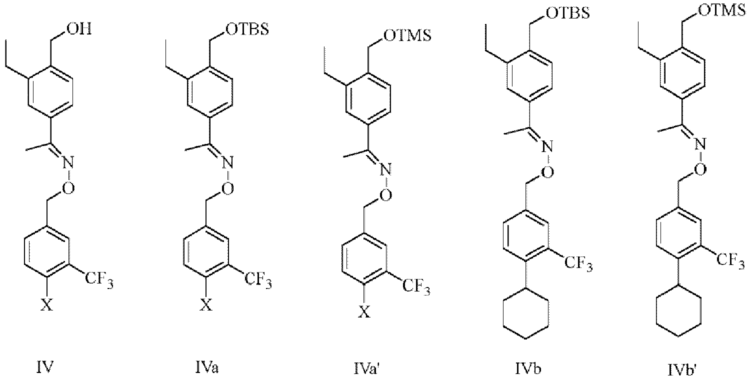 dans lequel R₂ et R₃, chacun indépendamment, représentent l'hydrogène, l'alkyle en C₁-C₄, l'halogène, le nitro ou R₂ et R₃ forment ensemble un anneau aliphatique ou aromatique de 5 à 7 membres, dans la préparation du Siponimod.
